# EUROPEAN PATENT SPECIFICATION

(11) **EP 2 782 521 B1**
(45) Date of publication and mention of the grant of the patent: **08.03.2017**
(21) Application number: 12850816.5
(22) Date of filing: 21.11.2012
(51) Int. Cl.: A61B 17/12

(54) **EMBOLIC DEVICE WITH SHAPED WIRE**
EMBOLISCHE VORRICHTUNG MIT EINEM PROFILDRAHT
DISPOSITIF EMBOLIQUE AYANT UN FIL MIS EN FORME

(30) Priority: 23.11.2011 US 201161563400 P; 09.07.2012 US 201261669645 P
(43) Date of publication of application: 01.10.2014
(73) Proprietor: Microvention, Inc., Tustin, CA (US)
(72) Inventor: BOWMAN, Heath, Trabuco Canyon, CA 92679 (US)
(74) Representative: KIPA AB
(86) International application number: PCT/US2012/066429
(87) International publication number: WO 2013/078438

(56) References cited:
- WO-A1-2011/053555
- WO-A2-02/13707
- US-A- 6 156 046
- US-A1- 2004 098 028
- US-A1- 2005 055 048
- US-A1- 2006 200 190
- US-A1- 2009 156 999
- US-A1- 2010 010 533
- US-A1- 2011 094 519
- US-B2- 7 833 175

## Description

### BACKGROUND OF THE INVENTION

The occlusion of body cavities, blood vessels, and other lumina by embolization is desired in a number of clinical situations. For example, the occlusion of fallopian tubes for the purposes of sterilization, and the occlusive repair of cardiac defects, such as a patent foramen ovale, patent ductus arteriosis, and left atrial appendage, and atrial septal defects. The function of an occlusion device in such situations is to substantially block or inhibit the flow of bodily fluids into or through the cavity, lumen, vessel, space, or defect for the therapeutic benefit of the patient.

The embolization of blood vessels is also desired in a number of clinical situations. For example, vascular embolization has been used to control vascular bleeding, to occlude the blood supply to tumors, and to occlude vascular aneurysms, particularly intracranial aneurysms. In recent years, vascular embolization for the treatment of aneurysms has received much attention. Several different treatment modalities have been shown in the prior art. One approach that has shown promise is the use of thrombogenic microcoils. These microcoils may be made of biocompatible metal alloy(s) (typically a radio-opaque material such as platinum or tungsten) or a suitable polymer. Examples of microcoils are disclosed in the following patents: U.S. Pat. No. 4,994,069-Ritchart et al.; U.S. Pat. No. 5,133,731--Butler et al.; U.S. Pat. No. 5,226,911-Chee et al.; U.S. Pat. No. 5,312,415--Palermo; U.S. Pat. No. 5,382,259-Phelps et al.; U.S. Pat. No. 5,382,260--Dormandy, Jr. et al.; U.S. Pat. No. 5,476,472-6-Dormandy, Jr. et al.; U.S. Pat. No. 5,578,074--Mirigian; U.S. Pat. No. 5,582,619--Ken; U.S. Pat. No. 5,624,461--Mariant; U.S. Pat. No. 5,645,558--Horton; U.S. Pat. No. 5,658,308--Snyder; and U.S. Pat. No. 5,718,711--Berenstein et al.

A specific type of microcoil that has achieved a measure of success is the Guglielmi Detachable Coil ("GDC"), described in U.S. Pat. No. 5,122,136--Guglielmi et al. The GDC employs a platinum wire coil fixed to a stainless steel delivery wire by a solder connection. After the coil is placed inside an aneurysm, an electrical current is applied to the delivery wire, which electrolytically disintegrates the solder junction, thereby detaching the coil from the delivery wire. The application of current also creates a positive electrical charge on the coil, which attracts negatively-charged blood cells, platelets, and fibrinogen, thereby increasing the thrombogenicity of the coil. Several coils of different diameters and lengths can be packed into an aneurysm until the aneurysm is completely filled. The coils thus create and hold a thrombus within the aneurysm, inhibiting its displacement and its fragmentation.

A more recent development in the field of microcoil vaso-occlusive devices is exemplified in U.S. Pat. No. 6,299,619 to Greene, Jr. et al., U.S. Pat. No. 6,602,261 to Greene, Jr. et al., and co-pending U.S. Pat. Appl. No. 10/631,981 to Martinez; These patents disclose vaso-occlusive devices comprising a microcoil with one or more expansile elements disposed on the outer surface of the coil. The expansile elements may be formed of any of a number of expansile polymeric hydrogels, or alternatively, environmentally-sensitive polymers that expand in response to a change in an environmental parameter (e.g., temperature or pH) when exposed to a physiological environment, such as the blood stream.

US20110094519 discloses a contraceptive device that may include an expandable implant. The implant includes hydrogel to provide substantially immediate sterilization, and tissue in-growth fibers to provide permanent sterilization.

WO2011053555 discloses devices for occlusion of body cavities, such as the embolization of vascular aneurysms and the like.

US6156046 discloses a system having a catheter, a torque member, a removal mechanism, and a guidewire.

WO0213707 discloses vaso-occlusive devices with selectively flattened surfaces.

### SUMMARY OF THE INVENTION

The invention is defined in the claims. A vaso-occlusive device comprises a carrier member, one or more novel expansile elements, and a combination thereof. Generally, the expansile element or elements comprise an expansile polymer. The carrier member may be used to assist the delivery of the expansile element by providing a structure that, in some embodiments, allows coupling to a delivery mechanism and, in some embodiments, enhances the radiopacity of the device.

In one embodiment, the carrier member may have a non-round, cross sectional shape. For example, the wire of the carrier member may have an oval, half circle, halve oval, double-D, or arc shape.

In one embodiment, the expansile polymer is an environmentally sensitive polymeric hydrogel, such as that described in U.S. Patent No. 6,878,384, issued April 12, 2005 to Cruise et al. In another embodiment, the expansile polymer is a novel hydrogel comprised of sodium acrylate and a poly(ethylene glycol) derivative. In another embodiment, the expansile polymer is a hydrogel comprising a Pluronics^{®} derivative.

In one embodiment, the expansile polymer is a novel hydrogel that has ionizable functional groups and is made from macromers. The macromers may be non-ionic and/or ethylenically unsaturated.

In one embodiment, the macromers may have a molecular weight of about 400 to about 35,000 grams/mole. In another embodiment the macromers may have a molecular weight of about 5,000 to about 15,000 grams/mole. In yet another embodiment the macromers may have a molecular weight of about 7,500 to about 12,000 grams/mole. In one embodiment the macromers have a molecular weight of 8,000 grams/mole.

In one embodiment, the hydrogel may be made of polyethers, polyurethanes, derivatives thereof, or combinations thereof. In another embodiment, the ionizable functional groups may comprise basic groups (e.g., amines, derivatives thereof, or combinations thereof) or acidic groups (e.g., carboxylic acids, derivatives thereof, or combinations thereof). If the ionizable functional groups comprise basic groups, the basic groups may be deprotonated at pHs greater than the pKa or protonated at pHs less than the pKa of the basic groups. If the ionizable functional groups comprise acidic groups, the acidic groups may be protonated at pHs less than the pKa or de-protonated at pHs greater than the pKa of the acidic groups.

In one embodiment, the macromers may comprise vinyl, acrylate, acrylamide, or methacrylate derivatives of poly(ethylene glycol), or combinations thereof. In another embodiment, the macromer may comprise poly(ethylene glycol) di-acrylamide. In another embodiment, the hydrogel is substantially free, more preferably free of unbound acrylamide.

In one embodiment, the macromers may be cross-linked with a compound that contains at least two ethylenically unsaturated moities. Examples of ethylenically unsaturated compounds include N, N'-methylenebisacrylamide, derivatives thereof, or combinations thereof. In another embodiment, the hydrogel may be prepared using a polymerization initiator. Examples of suitable polymerization initiators comprise N,N,N',N'-tetramethylethylenediamine, ammonium persulfate, azobisisobutyronitrile, benzoyl peroxides, derivatives thereof, or combinations thereof. The polymerization initiator may be soluble in aqueous or organic solvents. For example, azobisisobutyronitrile is not water soluble; however, water soluble derivatives of azobisisobutyronitrile, such as 2,2'-azobis(2-methylproprionamidine) dihydrochloride, are available. In another embodiment, the hydrogel may be substantially non-resorbable, non-degradable or both, at physiological conditions.

One example comprises a method for preparing an environmentally-responsive hydrogel for implantation in an animal. The method includes combining at least one, preferably non-ionic, macromer with at least one ethylenically unsaturated moiety, at least one macromer or monomer having at least one ionizable functional group and at least one ethylenically unsaturated moiety, at least one polymerization initiator, and at least one solvent to form a hydrogel. The solvent may include aqueous or organic solvents, or combinations thereof. In another embodiment, the solvent is water. Next, the hydrogel may be treated to prepare an environmentally-responsive hydrogel, preferably one that is responsive at physiological conditions. The ionizable functional group(s) may be an acidic group (e.g., a carboxylic acid, a derivative thereof, or combinations thereof) or a basic group (e.g., an amine, derivatives thereof, or combinations thereof). If the ionizable functional group comprises an acidic group, the treating step may comprise incubating the hydrogel in an acidic environment to protonate the acidic groups. If the ionizable functional group comprises a basic group, the treating step may comprise incubating the hydrogel in a basic environment to deprotonate the basic groups. In certain embodiments, it is preferable that the acidic groups are capable of being de-protonated or, conversely, the basic groups are capable of being protonated, after implantation in an animal.

In one embodiment, the ethylenically unsaturated macromer may have a vinyl, acrylate, methacrylate, or acrylamide group; including derivatives thereof or combinations thereof. In another embodiment, the ethylenically unsaturated macromer is based upon poly(ethylene glycol), derivatives thereof, or combinations thereof. In another embodiment, the ethylenically unsaturated macromer is poly(ethylene glycol) di-acrylamide, poly(ethylene glycol) di-acrylate, poly(ethylene glycol) di-methacrylate, derivatives thereof, or combinations thereof. In another embodiment, the ethylenically unsaturated macromer is poly(ethylene glycol) di-acrylamide. The ethylenically unsaturated macromer may be used at a concentration of about 5% to about 40% by weight, more preferably about 20% to about 30% by weight. The solvent may be used at a concentration of about 20% to about 80% by weight.

In one embodiment, the combining step also includes adding at least one cross-linking agent comprising an ethylenically unsaturated compound. In certain embodiments of the present invention, a cross-linker may not be necessary. In other words, the hydrogel may be prepared using a macromer with a plurality of ethylenically unsaturated moieties. In another embodiment, the polymerization initiator may be a reduction-oxidation polymerization initiator. In another embodiment, the polymerization initiator may be N,N,N',N'-tetramethylethylenediamine, ammonium persulfate, azobisisobutyronitrile, benzoyl peroxides, 2,2'-azobis(2-methylproprionamidine) dihydrochloride, derivatives thereof, or combinations thereof. In another embodiment, the combining step further includes adding a porosigen.

In one embodiment, the ethylenically unsaturated macromer includes poly(ethylene glycol) di-acrylamide, the macromer or monomer or polymer with at least one ionizable group and at least one ethylenically unsaturated group includes sodium acrylate, the polymerization initiator includes ammonium persulfate and N,N,N,',N' tetramethylethylenediamine, and the solvent includes water.

In one embodiment, the ethylenically unsaturated macromer has a molecular weight of about 400 to about 35,000 grams/mole. In another embodiment, the ethylenically unsaturated macromer has a molecular weight of about 5,000 to about 15,000 grams/mole. In one embodiment, the ethylenically unsaturated macromer has a molecular weight of about 7,500 to about 12,000 grams/mole. In another embodiment, the environmentally-responsive hydrogel is substantially non-resorbable, or non-degradable or both at physiological conditions. In certain embodiments, the environmentally-responsive hydrogel may be substantially free or completely free of unbound acrylamide.

In one embodiment, the carrier member comprises a coil or microcoil made from metal, plastic, or similar materials. In another embodiment, the carrier member comprises a braid or knit made from metal, plastic, or similar materials. In another embodiment, the carrier member comprises a plastic or metal tube with multiple cuts or grooves cut into the tube.

In one embodiment, the expansile element is arranged generally co-axially within the carrier member. In another embodiment, a stretch resistant member is arranged parallel to the expansile element. In another embodiment, the stretch resistant member is wrapped, tied, or twisted around the expansile element. In another embodiment, the stretch resistant member is positioned within the expansile element. In another embodiment, the stretch resistant member is located within or partially surrounded by the expansile element.

In one embodiment, the device comprising the expansile element and carrier member are detachably coupled to a delivery system. In another embodiment, the device is configured for delivery by pushing or injecting through a conduit into a body.

In one embodiment, the expansile element is environmentally sensitive and exhibits delayed expansion when exposed to bodily fluids. In another embodiment, the expansile element expands quickly upon contact with a bodily fluid. In another embodiment, the expansile element comprises a porous or reticulated structure that may form a surface or scaffold for cellular growth.

In one embodiment, the expansile element expands to a dimension that is larger than the diameter of the carrier member in order to provide enhanced filling of the lesion. In another embodiment, the expansile element expands to a dimension equal to or smaller than the diameter of the carrier member to provide a scaffold for cellular growth, release of therapeutic agents such as pharmaceuticals, proteins, genes, biologic compounds such as fibrin, or the like.

### BRIEF DESCRIPTION OF THE DRAWINGS

**Fig. 1** is a perspective view showing one embodiment of the present invention prior to expansion of the expansile element;
**Fig. 2** is a perspective view showing a device similar to **Fig. 1** in an expanded state;
**Fig. 3** is a perspective view of an alternative embodiment of the present invention;
**Fig. 4** is a perspective view of an alternative embodiment wherein the carrier member comprises a fenestrated tube, braid or knit;
**Fig. 5** is a perspective view of an alternative embodiment incorporating a stretch resistant member running approximately parallel to the expansile element;
**Fig. 6** is a perspective view of an alternative embodiment incorporating a stretch resistant member approximately intertwined with the expansile element;
**Fig. 7** is a perspective view of an alternative embodiment wherein the expansile element has formed a loop or fold outside the carrier member.
**Fig. 8** is a perspective view of an alternative embodiment showing a device similar to those shown in **Fig.1** and **Fig. 2** wherein the expansile element is not expanded to a diameter larger than the carrier member.
**Fig. 9** is a side view of an embodiment showing a device similar to those shown in **Fig.1** and **Fig. 2****.**
**Fig. 10** is an exploded perspective view of the device of **Fig. 9****.**
**Fig. 11** is a side view of the device of **Fig. 9** connected to a delivery device.
**Fig. 12** is a side view of a preferred embodiment of an implant according to the present invention.
**Fig. 13** is a side view of a preferred embodiment of an implant according to the present invention.
**Fig. 14** is a cross sectional view of a carrier member according to the present invention.
**Fig. 15** is a cross sectional view of a carrier member according to the present invention.
**Fig. 16** is a cross sectional view of a carrier member according to the present invention.
**Fig. 17** is a cross sectional view of a carrier member according to the present invention.
**Fig. 18** is a cross sectional view of the carrier member of Fig. 14 according to the present invention.
**Fig. 19** is a side view of a carrier member according to the present invention.
**Fig. 20** is a side view of a carrier member according to the present invention.
**Fig. 21** is a side view of a carrier member according to the present invention.
**Figs. 22-24** are a side view of a carrier member according to an alternative embodiment of the present invention

### DESCRIPTION OF THE INVENTION

As used herein, the term "macromer" refers to a large molecule containing at least one active polymerization site or binding site. Macromers have a larger molecular weight than monomers. For example, an acrylamide monomer has a molecular weight of about 71.08 grams/mole whereas a poly(ethylene glycol) di-acrylamide macromer may have a molecular weight of about 400 grams/mole or greater. Preferred macromers are non-ionic, i.e. they are uncharged at all pHs.

As used herein, the term "environmentally responsive" refers to a material (e.g., a hydrogel) that is sensitive to changes in environment including but not limited to pH, temperature, and pressure. Many of the expansile materials suitable for use in the present invention are environmentally responsive at physiological conditions.

As used herein, the term "non-resorbable" refers to a material (e.g., a hydrogel) that cannot be readily and/or substantially degraded and/or absorbed by bodily tissues.

As used herein, the term "unexpanded" refers to the state at which a hydrogel is substantially not hydrated and, therefore, not expanded.

As used herein, the term "ethylenically unsaturated" refers to a chemical entity (e.g., a macromer, monomer or polymer) containing at least one carbon-carbon double bond.

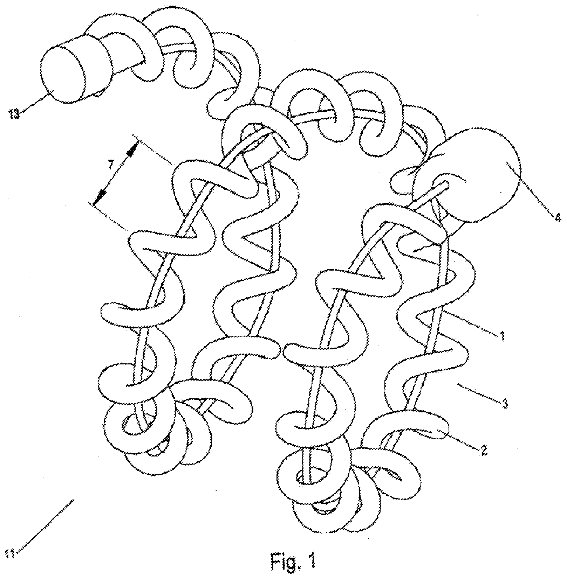

Referring to **Fig. 1-8****,** the device comprises an expansile element 1 and a carrier member 2. The expansile element 1 may be made from a variety of suitable biocompatible polymers. In one embodiment, the expansile element 1 is made of a bioabsorbable or biodegradable polymer, such as those described in U.S. Patent Nos. 7,070,607 and 6,684,884. In another embodiment, the expansile element 1 is made of a soft conformal material, and more preferably of an expansile material such as a hydrogel.

In one embodiment, the material forming the expansile element 1 is an environmentally responsive hydrogel, such as that described in U.S. Patent No. 6,878,384. Specifically, the hydrogels described in U.S. Patent No. 6,878,384 are of a type that undergoes controlled volumetric expansion in response to changes in such environmental parameters as pH or temperature. These hydrogels are prepared by forming a liquid mixture that contains (a) at least one monomer and/or polymer, at least a portion of which is sensitive to changes in an environmental parameter; (b) a cross-linking agent; and (c) a polymerization initiator. If desired, a porosigen (e.g., NaCl, ice crystals, or sucrose) may be added to the mixture, and then removed from the resultant solid hydrogel to provide a hydrogel with sufficient porosity to permit cellular ingrowth. The controlled rate of expansion is provided through the incorporation of ethylenically unsaturated monomers with ionizable functional groups (e.g., amines, carboxylic acids). For example, if acrylic acid is incorporated into the cross-linked network, the hydrogel is incubated in a low pH solution to protonate the carboxylic acid groups. After the excess low pH solution is rinsed away and the hydrogel dried, the hydrogel can be introduced through a microcatheter filled with saline at physiological pH or with blood. The hydrogel cannot expand until the carboxylic acid groups deprotonate. Conversely, if an amine-containing monomer is incorporated into the cross-linked network, the hydrogel is incubated in a high pH solution to deprotonate amines. After the excess high pH solution is rinsed away and the hydrogel dried, the hydrogel can be introduced through a microcatheter filled with saline at physiological pH or with blood. The hydrogel cannot expand until the amine groups protonate.

In another embodiment, the material forming the expansile element 1 may be an environmentally responsive hydrogel, similar to those described in U.S. Patent No. 6,878,384; however, an ethylenically unsaturated, and preferably non-ionic, macromer replaces or augments at least one monomer or polymer. The Applicants surprisingly have discovered that hydrogels prepared in accordance with this embodiment can be softer and/or more flexible in their unexpanded state than those prepared in accordance with U.S. Patent No. 6,878,384. The Applicants also have discovered that ethylenically unsaturated and non-ionic macromers (e.g., poly(ethylene glycol) and derivatives thereof) may be used not only to prepare a softer unexpanded hydrogel; but, in combination with monomers or polymers containing ionizable groups, one that also may be treated to be made environmentally responsive. The surprising increase in unexpanded flexibility enables the hydrogels to be, for example, more easily deployed in an animal or deployed with reduced or no damage to bodily tissues, conduits, cavities, etceteras.

The hydrogels prepared from non-ionic macromers in combination with monomers or polymers with ionizable functional groups still are capable of undergoing controlled volumetric expansion in response to changes in environmental parameters. These hydrogels may be prepared by combining in the presence of a solvent: (a) at least one, preferably non-ionic, macromer with a plurality of ethylenically unsaturated moieties; (b) a macromer or polymer or monomer having at least one ionizable functional group and at least one ethylenically unsaturated moiety; and (c) a polymerization initiator. It is worthwhile to note that with this type of hydrogel, a cross-linking agent may not be necessary for cross-linking since, in certain embodiments, the components selected may be sufficient to form the hydrogel. As hereinbefore described, a porosigen may be added to the mixture and then removed from the resultant hydrogel to provide a hydrogel with sufficient porosity to permit cellular ingrowth.

The non-ionic macromer-containing hydrogels' controlled rate of expansion may be provided through the incorporation of at least one macromer or polymer or monomer having at least one ionizable functional group (e.g., amine, carboxylic acid). As discussed above, if the functional group is an acid, the hydrogel is incubated in a low pH solution to protonate the group. After the excess low pH solution is rinsed away and the hydrogel dried, the hydrogel can be introduced through a microcatheter, preferably filled with saline. The hydrogel cannot expand until the acid group(s) deprotonates. Conversely, if the functional group is an amine, the hydrogel is incubated in a high pH solution to deprotonate the group. After the excess high pH solution is rinsed away and the hydrogel dried, the hydrogel can be introduced through a microcatheter, preferably filled with saline. The hydrogel cannot expand until the amine(s) protonates.

More specifically, in one embodiment, the hydrogel is prepared by combining at least one non-ionic macromer having at least one unsaturated moiety, at least one macromer or monomer or polymer having at least one ionizable functional group and at least one ethylenically unsaturated moiety, at least one polymerization initiator, and a solvent. Optionally, an ethylenically unsaturated cross-linking agent and/or a porosigen also may be incorporated. In one embodiment, concentrations of the non-ionic macromers in the solvent range from about 5% to about 60% (w/w). In another embodiment, concentrations of the non-ionic macromers in the solvent range from about 20% to about 30% (w/w). In one embodiment, concentrations of the non-ionic macromers in the solvent range are about 25% (w/w). In one embodiment the non-ionic macromer is poly(ethylene glycol), its derivatives, and combinations thereof. Derivatives include, but are not limited to, poly(ethylene glycol) di-acrylamide, poly(ethylene glycol) di-acrylate, and poly(ethylene glycol) dimethacrylate. Poly(ethylene glycol) di-acrylamide is a preferred derivative of poly(ethylene glycol) and has a molecular weight ranging from about 8,500 grams/mole to about 12,000 grams/mole. The macromer may have less than 20 polymerization sites, more preferably less than 10 polymerization sites, more preferably about five or less polymerization sites, and more preferably from about two to about four polymerization sites. Poly(ethylene glycol) di-acrylamide has two polymerization sites.

Preferred macromers or polymers or monomers having at least one ionizable functional group include, but are not limited to compounds having carboxylic acid or amino moieties or, derivatives thereof, or combinations thereof. Sodium acrylate is a preferred ionizable functional group-containing compound and has a molecular weight of 94.04 g/mole. In one embodiment, concentrations of the ionizable macromers or polymers or monomers in the solvent range from about 5% to about 60% (w/w) In another embodiment, concentrations of the ionizable macromers or polymers or monomers in the solvent range from about 20% to about 30% (w/w). In one embodiment, concentrations of the ionizable macromers or polymers or monomers in the solvent are about 27% (w/w). In some embodiments, at least about 10%-50% of the ionizable macromers or polymers or monomers selected should be pH sensitive. In other embodiments at least about 10%-30% of the ionizable macromers or polymers or monomers selected should be pH sensitive. In one embodiment no free acrylamide is used in the macromer-containing hydrogels.

When used, the cross-linking agent may be any multifunctional ethylenically unsaturated compound, preferably N, N'-methylenebisacrylamide. If biodegradation of the hydrogel material is desired, a biodegradable cross-linking agent may be selected. The concentrations of the cross-linking agent in the solvent should be less than about 1% w/w, and preferably less than about 0.1 % (w/w).

As described above, if a solvent is added, it may be selected based on the solubilities of the macromer(s) or monomer(s) or polymer(s), cross-linking agent, and/or porosigen used. If a liquid macromer or monomer or polymer solution is used, a solvent may not be necessary. A preferred solvent is water, but a variety of aqueous and organic solvents may be used. In one embodiment, concentrations of the solvent range from about 20% to about 80% (w/w). In another embodiment, concentrations of the solvent range from about 40% to about 60% (w/w).

Crosslink density may be manipulated through changes in the macromer or monomer or polymer concentration, macromer molecular weight, solvent concentration and, when used, cross-linking agent concentration. As described above, the hydrogel may be cross-linked via reduction-oxidation, radiation, and/or heat. A preferred type of polymerization initiator is one that acts via reduction-oxidation. Suitable polymerization initiators include, but are not limited to, N,N,N',N'-tetramethylethylenediamine, ammonium persulfate, azobisisobutyronitrile, benzoyl peroxides, 2,2'-azobis(2-methylpropionamidine) dihydrochloride, derivatives thereof, or combinations thereof. A combination of ammonium persulfate and N,N,N',N'-tetramethylethylenediamine is a preferred polymerization initiator for use in the macromer containing embodiments.

After polymerization is complete, the hydrogels may be washed with water, alcohol or other suitable washing solution(s) to remove any porosigen(s), any unreacted, residual macromer(s), monomer(s), and polymer(s) and any unincorporated oligomers. Preferably this is accomplished by initially washing the hydrogel in distilled water.

Porosity may be imparted into the solid hydrogel through the use of porosigens such as sodium chloride, ice crystals, or sucrose. Polymerization of the monomer solution around the solid particles in suspension and subsequent removal of the solid particles from the hydrogel can provide a hydrogel with sufficient porosity to permit cellular ingrowth. A preferred porosigen is sodium chloride with particles less than 10 micrometer (microns) in diameter. Preferred sodium chloride concentrations in the monomer solution range from 0.2 to 0.4 g sodium chloride per g monomer solution.

The hydrogels may be made environmentally-responsive by protonating or deprotonating the ionizable functional groups present on the hydrogel network, as discussed above. Once the hydrogel has been prepared and, if needed, washed, the hydrogel may be treated to make the hydrogel environmentally-responsive. For hydrogel networks where the ionizable functional groups are carboxylic acid groups, the hydrogel is incubated in a low pH solution. The free protons in the solution protonate the carboxylic acid groups on the hydrogel network. The duration and temperature of the incubation and the pH of the solution influence the amount of control on the expansion rate. In general, the duration and temperature of the incubation are directly proportional to the amount of expansion control, while the incubation solution pH is inversely proportional thereto.

It has been determined that incubation solution water content also affects expansion control. In this regard, higher water content enables greater hydrogel expansion and is thought to increase the number of protonation-accessible carboxylic acid groups. An optimization of water content and pH is required for maximum control on expansion rate. Expansion control, among other things, has an effect on device positioning/repositioning time. Typically, a positioning/repositioning time of about 0.1 to about 30 minutes is preferred for hydrogel devices in accordance with the present embodiments.

After incubation, the excess treating solution is washed away and the hydrogel material is dried. A hydrogel treated with the low pH solution has been observed to dry down to a smaller dimension than an untreated hydrogel. This effect is desirable since devices containing these hydrogels may be delivered through a microcatheter.

For hydrogel networks where the ionizable functional groups are amine groups, the hydrogel is incubated in a high pH solution. Unlike carboxylic acid functional groups, deprotonation occurs on the amine groups of the hydrogel network at high pH. Aside from incubation solution pH, the incubation is carried out similarly to that of the carboxylic acid containing hydrogels. In other words, the duration and temperature of the incubation and the pH of the solution are directly proportional to the amount of expansion control. After incubation is concluded, the excess treating solution is washed away and the hydrogel material is dried.

In a preferred embodiment, the expansile element 1 is an expansile hydrogel comprised of (a) at least one, preferably non-ionic, ethylenically unsaturated macromer or monomer or polymer having at least two cross-linkable groups; (b) at least one monomer and/or polymer which has at least one cross-linkable groups, and at least one moiety that is sensitive to changes in an environmental parameter; and (c) a polymerization initiator. In some embodiments, the monomers and polymers may be water soluble, while in other embodiments they may be non-water soluble. Suitable polymers for component (a) include poly(ethylene glycol), poly(ethylyene oxide), poly(vinyl alcohol), poly(propylene oxide), poly(propylene glycol), poly(ethylene oxide)-co-poly(propylene oxide), poly(vinyl pyrrolidinone), poly(amino acids), dextrans, poly(ethyloxazoline), polysaccharides, proteins, glycosaminoglycans, and carbohydrates, and derivatives thereof. The preferred polymer is poly(ethylene glycol) (PEG), especially for component (a). Alternatively, polymers that biodegrade partly or completely may be utilized.

One embodiment comprises combining in the presence of a solvent (a) about 5% to about 50% of a non-ionic, ethylenically unsaturated macromer or monomer or polymer; (b) about 5% to about 60% of an ethylenically unsaturated monomer or polymer with at least one ionizable functional group; and, (c) a polymerization initiator. Suitable ionizable, ethylenically unsaturated monomers include acrylic acid and methacrylic acid, as well as derivatives thereof. One suitable monomer having at least one ionizable functional group is sodium acrylate. Suitable macromers with two ethylenically unsaturated moities include poly(ethylene glycol) di-acrylate and poly(ethylene glycol) di-acrylamide, and poly(ethylene glycol) di-acrylamide, which have molecular weights ranging between 400 and 30,000 grams/mole. The use of macromers with a plurality of ethylenically unsaturated groups permits the elimination of the cross-linker, as the cross-linker functions are performed by the multi-functional polymer. In one embodiment, the hydrogel comprises, about 5% to about 60% sodium acrylate, about 5% to about 50% poly(ethylene glycol) di-acrylamide.

A sodium acrylate/ poly(ethylene glycol) di-acrylamide hydrogel is used to enhance the mechanical properties of the previously-described environmentally responsive hydrogel. Since a sodium acrylate/poly(ethylene glycol) di-acrylamide hydrogel is softer than a sodium acrylate/acrylamide hydrogel (e.g., the one utilized in *Hydrogel Embolic System (HES)* made by MicroVention, Aliso Viejo, CA), devices incorporating it may be more flexible. Due to the relative stiffness of the HES, MicroVention recommends pre-softening the device by soaking in warm fluid or steaming the implant. In addition, devices made from acrylamide are relatively straight before pre-softening because the stiffness of the acrylamide-based hydrogel prevents the carrier member (for the HES, a microcoil) from assuming its secondary configuration. Devices made from a sodium acrylate/poly(ethylene glycol) di-acrylamide hydrogel may not require pre-softening techniques such as soaking in warm fluid such as saline or blood or exposure to steam in order to form into a secondary configuration heat-set into the carrier member 2 or a similar carrier member. Thus, in embodiments comprising, for example, sodium acrylate and poly(ethylene glycol) di-acrylamide, a substantially continuous length of hydrogel disposed either within the lumen 3 of the carrier member 2 as shown in, for example, **Fig. 1** or on a carrier element such as those shown in the Martinez '981 application or Greene '261, will form into the secondary configuration pre-formed into the carrier member without pre-treatment (e.g. exposure to steam, fluid, or blood). This makes the device easier to use because it allows elimination of the pre-treatment step and the device may be safer when deployed into the patient because a softer device is less likely to cause damage to the lesion.

### Examples

3 g of acrylamide, 1.7 g of acrylic acid, 9 mg of bisacrylamide, 50 mg of N,N,N',N'-tetramethylethylenediamine, 15 mg of ammonium persulfate, and 15.9 g water were combined and polymerized in a 0.508 mm (0.20 inch) tube. The tubularized polymer was removed from the tubing to prepare Hydrogel 1 in accordance with U.S. Patent No. 6,878,384.

4.6 g of poly(ethylene glycol) diacrylamide, 3.3 g of sodium acrylate, 100 mg of N,N,N',N'-tetramethylethylenediamine, 25 mg of ammonium persulfate, and 15.9 g water were combined and polymerized in a 0.508 mm (0.20 inch) tube. The tubularized polymer was removed from the tubing to prepare Hydrogel 2, in accordance with a macromer-containing hydrogel embodiment.

A large platinum microcoil for the above examples has a 0.3556 mm (0.014 inch) outer diameter and a 0.0635 mm (0.0025 inch) filar. A small platinum microcoil has a 0.254 mm (0.010 inch) outer diameter and a 0.508 mm (0.20 inch) filar.

8.3 g of poly(ethylene glycol) diacrylamide, 9.0 g of sodium acrylate, 155 mg of N,N,N',N'-tetramethylethylenediamine, 20 mg of ammonium persulfate, and 15.9 g water were combined and polymerized in a 0.635 mm (0.025 inch) tube. The tubularized polymer was removed from the tubing to prepare Hydrogel 3, in accordance with a macromer-containing hydrogel embodiment.

The Hydrogel 3 is distinct from the Hydrogel 1 and 2 examples. The Hydrogel 3 has a reduced stiffness relative to Hydrogel 1 and it further does not require pretreatment prior to use. Such pretreatment can sometimes require soaking in warm fluid or steaming to achieve a desired flexibility. Hydrogel 3 also allows for increased expansion compared with Hydrogel 2.

In another embodiment, monomers are used to impart moieties to the expansile element 1 that are suitable for coupling bioactive compounds, for example anti-inflammatory agents such as corticosteroids (e.g. prednisone and dexamethasone); or vasodilators such as nitrous oxide or hydralazine; or anti-thrombotic agents such as aspirin and heparin; or other therapeutic compounds, proteins such as mussel adhesive proteins (MAPs), amino acids such as 3-(3,4-dihydroxyphenyl)-L-alanine (DOPA), genes, or cellular material; see U.S Patent 5,658,308, WO 99/65401, Polymer Preprints 2001,42(2), 147 Synthesis and Characterization of Self-Assembling Block Copolymers Containing Adhesive Moieties by Kui Hwang et. al., and WO 00/27445 the disclosures of which are hereby incorporated by reference. Examples of moieties for incorporation into hydrogel materials include, but are not limited to, hydroxyl groups, amines, and carboxylic acids.

In another embodiment, the expansile element 1 may be rendered radiopaque by incorporation of monomers and/or polymers containing, for example, iodine, or the incorporation of radiopaque metals such as tantalum and platinum.

In some embodiments, the carrier member 2 is a flexible, elongate structure. Suitable configurations for the carrier member 2 include helical coils, braids, and slotted or spiral-cut tubes. The carrier member 2 may be made of any suitable biocompatible metal or polymer such as platinum, tungsten, PET, PEEK, Teflon, Nitinol, Nylon, steel, and the like. The carrier member may be formed into a secondary configuration such as helix, box, sphere, flat rings, J-shape, S-shape or other complex shape known in the art. Examples of appropriate shapes are disclosed in Horton 5,766,219; Schaefer Appl. No. 10/043,947; and Wallace 6,860,893. all hereby incorporated by reference.

As previously described, some embodiments may comprise polymers that are sufficiently soft and flexible that a substantially continuous length of the expansile element 1 will form into a secondary configuration similar to the configuration originally set into the carrier member 2 without pre-softening the device or exposing it to blood, fluid, or steam.

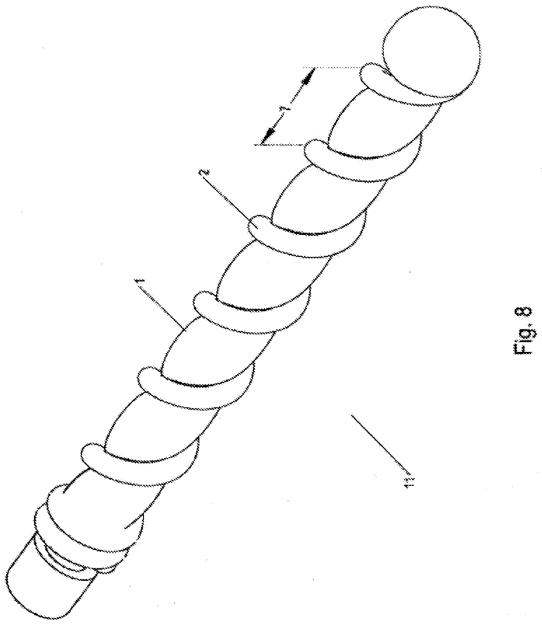

In some embodiments, the carrier member 2 incorporates at least one gap 7 that is dimensioned to allow the expansile element 1 to expand through the gap (one embodiment of this configuration is shown in **Figs. 1-2****).** In other embodiments, the carrier member 2 incorporates at least one gap 7 that allows the expansile element 1 to be exposed to bodily fluids, but the expansile element 1 does not necessarily expand through the gap (one embodiment of this configuration is shown in **Fig. 8****).** In other embodiments, no substantial gap is incorporated into the carrier member 2. Rather, fluid is allowed to infiltrate through the ends of the device or is injected through a lumen within the delivery system and the expansile element 1 expands and forces its way through the carrier member 2.

In one embodiment shown in **Fig. 1****,** the expansile element 1 comprises an acrylamide or poly(ethylene glycol)-based expansile hydrogel. The carrier member 2 comprises a coil. At least one gap 7 is formed in the carrier member 2. The expansile element 1 is disposed within the lumen 3 defined by the carrier member 2 in a generally coaxial configuration. A tip 4 is formed at the distal end of the device 11 by, for example, a laser, solder, adhesive, or melting the hydrogel material itself. The expansile element 1 may run continuously from the proximal end to the distal end, or it may run for a portion of the device then terminate before reaching the distal or proximal end, or both.

As an example, in one embodiment the device is dimensioned to treat a cerebral aneurysm. Those skilled in the art will appreciate that the dimensions used in this example could be re-scaled to treat larger or smaller lesions. In this embodiment, the expansile element 1 is about 0.1524-1.1778 mm (0.006" 0.007") before expansion and about 0.508 mm (0.02") after expansion. The expansile element is, for example, approximately 52% sodium acrylate, 48% poly(ethylene glycol) di-acrylamide with a molecular weight about 8000 grams/mole. About 0.4 g/g sodium chloride (about 10 micrometer (microns) particle size) is used as a porosigen and about 0.6 mg/mL ammonium persulfate and 7 mg/mL tetramethylethylene diamine is used as an initiator. The carrier member 2 in this embodiment is a microcoil in the range of about 0.3048-0.3175 mm (0.012"-0.0125") in diameter and has a filar between about 0.0508-0.05715 mm (0.002"-0.0225"). In one embodiment, the carrier member 2 comprises at least one gap 7 between 1 to 3 filar sizes long. In another embodiment, the carrier member 2 comprises at least one gap 7 that is about 2 filars long. In one embodiment the size of the gap 7 is between about 0.0381 mm (0.0015 inches) and 0.1905 mm (0.0075 inches) long. In another embodiment, the size of the gap 7 is between 0.05715 mm (0.00225 inches) and 0.1905 mm (0.00750 inches) long.

A coupler 13 is placed near the proximal end to allow the implant 11 to be detachably coupled to a delivery system or pushed or injected through a catheter. Examples of delivery systems are found in co-pending Appl. No. 11/ 212,830 to Fitz, US6,425,893 to Guglielmi, US4,994,069 to Ritchart, US6,063,100 to Diaz, and US5,690,666 to Berenstein.

In this embodiment, the implant 11 is constructed by formulating and mixing the hydrogel material as previously described in order to form the expansile element 1. The carrier member 2 is wound around a helical or complex form, and then heat-set by techniques known in the art to form a secondary diameter ranging from 0.5 mm to 30 mm and a length ranging from 5 mm to 100 cm. After processing, washing, and optional acid treatment, the expansile element 1 is threaded through the lumen 3 of the carrier member 2. The distal end of the expansile element 1 is then tied, for example by forming a knot, to the distal end of the carrier member 2. Adhesive, such as UV curable adhesive or epoxy, may be used to further enhance the bond between the expansile element 1 and the carrier member 2 and to form the distal tip 4. Alternatively, the tip may be formed by, for example, a laser weld or solder ball.

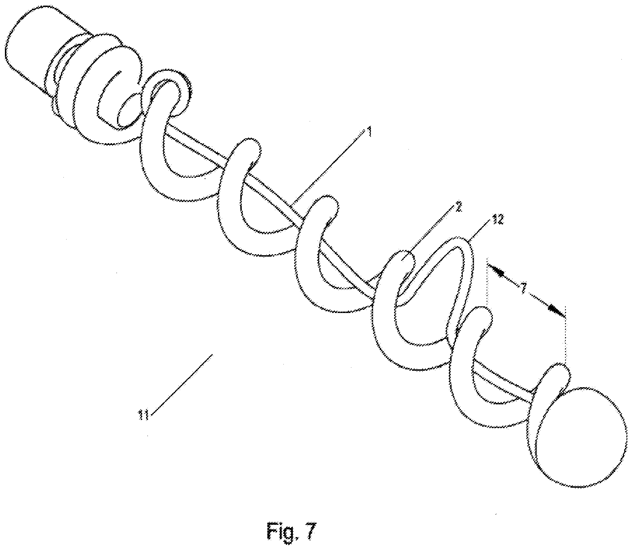

In some embodiments, the size of the gap 7 and the ratio of expansion, loops or folds 12 may form as shown in **Fig. 7** as the expansile element 1 expands. It is desirable to prevent these loops or folds 12 from forming. This can be done by stretching the expansile element 1 either before placing it within the carrier member 2 or after the distal end of the expansile element 1 is secured to the carrier member 2. For example, once the distal end of the expansile element 1 is secured to the carrier member 2, the expansile element 1 is stretched such that its initial diameter of 0.254 mm (0.010") is reduced to between about 0.1524-0.1778 mm (0.006"-0.007") before placing it within the carrier member 2. After stretching, the expansile element 1 may be trimmed to match the length of the carrier member 2 and then bonded near the proximal end of the carrier member 2 by, for example, tying a knot, adhesive bonding, or other techniques known in the art.

Once the implant 11 has been constructed, it is attached to a delivery system previously described by methods known in the art. The device may also be exposed to, for example, e-beam or gamma radiation to cross-link the expansile element 1 and to control its expansion. This is described in U.S. Patent No. 6,537,569 which is assigned to the assignee of this application and hereby incorporated by reference.

Previously, the secondary dimensions of prior devices (e.g. HES) are generally sized to a dimension 1-2 mm smaller than the dimension (i.e. volume) of the treatment site due to the relative stiffness of these devices. The increased flexibility and overall design of the implant 11 allows the secondary shape of the implant 11 to be sized to a dimension approximately the same size as the treatment site, or even somewhat larger. This sizing further minimizes the risk of the implant moving in or slipping out of the treatment site.

Prior implant devices, such as the HES device, currently provide the user with about 5 minutes of repositioning time. However, the implant 11 increases the length of repositioning time. In some embodiments, the repositioning time during a procedure can be increased to about 30 minutes. In this respect, the user is provided with a longer repositioning time to better achieve a desired implant configuration

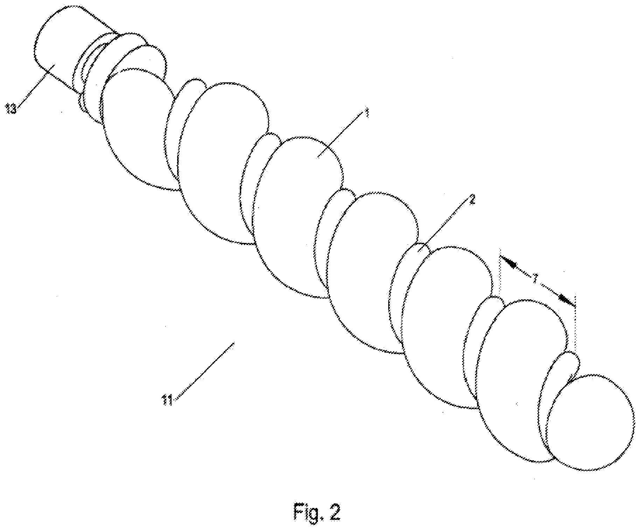

**Fig. 2** shows an implant 11 similar to that shown in **Fig. 1** after the expansile element 1 has expanded through the gap 7 to a dimension larger than the carrier member 2.

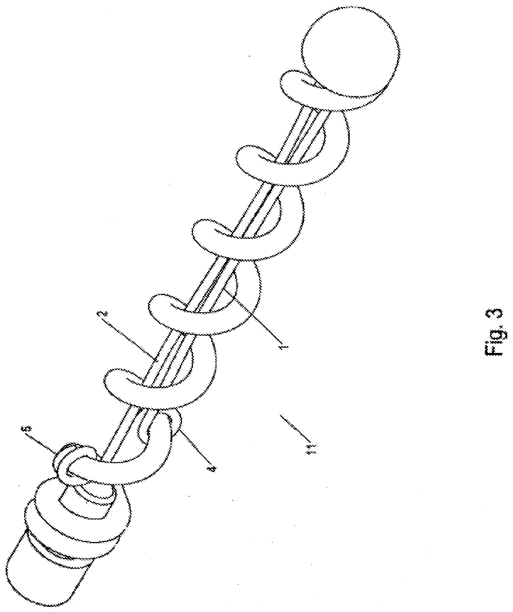

**Fig. 3** shows an implant 11 wherein multiple expansile elements 1 run somewhat parallel to each other through the carrier member 2. In one embodiment, this configuration is constructed by looping a single expansile element 1 around the tip 4 of the implant 11 and tying both ends of the expansile element 1 to the proximal end of the carrier member 2. In another embodiment, multiple strands of the expansile element 1 may be bonded along the length of the carrier member 2. The construction of these embodiments may also comprise stretching the expansile element 1 as previously described and/or forming gaps in the carrier member 2.

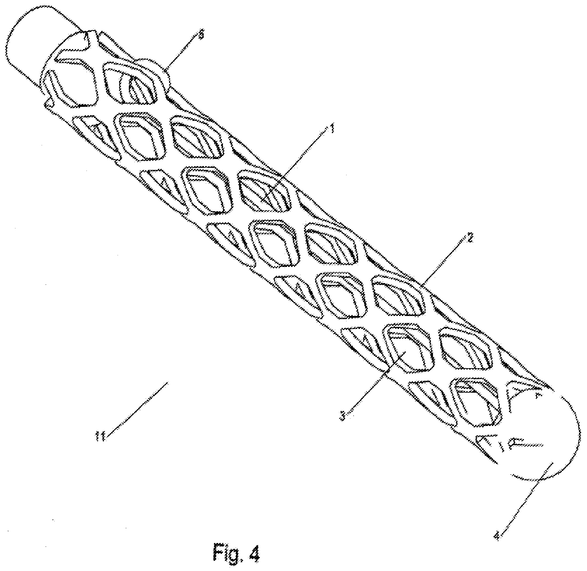

**Fig. 4** shows an embodiment wherein the implant 11 comprises a non-coil carrier member 2. In one embodiment, the carrier member 2 is formed by cutting a tube or sheet of plastic such as polyimide, nylon, polyester, polyglycolic acid, polylactic acid, PEEK, Teflon, carbon fiber or pyrolytic carbon, silicone, or other polymers known in the art with, for example; a cutting blade, laser, or water jet in order to form slots, holes, or other fenestrations through which the expansile element 1 may be in contact with bodily fluids. The plastic in this embodiment may also comprise a radiopaque agent such as tungsten powder, iodine, or barium sulfate. In another embodiment, the carrier member 2 is formed by cutting a tube or sheet of metal such as platinum, steel, tungsten, Nitinol, tantalum, titanium, chromium-cobalt alloy, or the like with, for example; acid etching, laser, water jet, or other techniques known in the art. In another embodiment, the carrier member 2 is formed by braiding, knitting, or wrapping metallic or plastic fibers in order to form fenestrations.

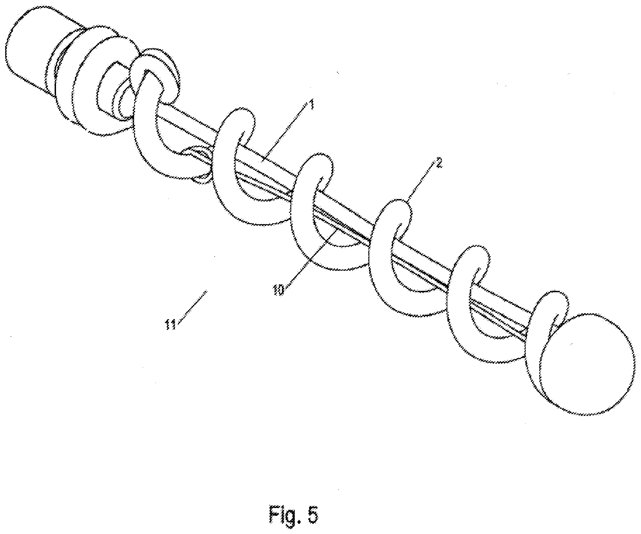

**Fig. 5** shows an implant 11 comprising a carrier member 2, an expansile element 1, and a stretch resistant member 10. The stretch resistant member 10 is used to prevent the carrier member 2 from stretching or unwinding during delivery and repositioning. The stretch resistant member 10 may be made from a variety of metallic or plastic fibers such as steel, Nitinol, PET, PEEK, Nylon, Teflon, polyethylene, polyolefin, polyolefin elastomer, polypropylene, polylactic acid, polyglycolic acid, and various other suture materials known in the art. Construction of the implant 11 may be by attaching the ends of the stretch resistant member 10 to the ends of the carrier member 2 as described by US6,013,084 to Ken and US5,217,484 to Marks. Alternatively, the distal end of the stretch resistant member 10 may be attached near the distal end of the carrier member 2 and the proximal end to the stretch resistant member 10 attached to the delivery system as described in co-pending Appl. No. 11/ 212,830 to Fitz.

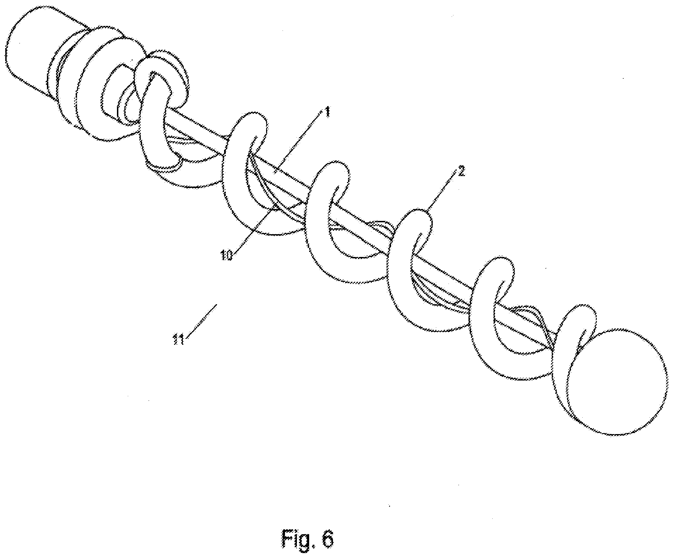

**Fig. 6** is an alternative embodiment comprising a stretch resistant member 10 wrapped around, tied to, or intertwined with the expansile element 1. This may occur over the length of the expansile element 1, or the wrapping or tying may be in only one area to facilitate bonding the expansile element 1 to the carrier element 2 by using the stretch resistant member 10 as a bonding element.

**Fig. 7** shows a loop or fold 12 of the expansile element 1 protruding outside the carrier element 2. In some embodiments, it may be desirable to avoid this condition by, for example, stretching the expansile element 1 as previously described. This would be done, for example, in embodiments configured for delivery through a small microcatheter to prevent the implant 11 from becoming stuck in the microcatheter during delivery. In other embodiments, slack may be added to the expansile element 1 so that the loop or fold will be pre-formed into the implant 11. This would be done in embodiments where, for example, a larae amount of volumetric filling was necessary because the loops or folds would tend to increase the total length of the expansile element 1.

**Fig. 8** shows an embodiment wherein the expansile element 1 is configured to expand to a dimension larger than its initial dimension, but smaller than the outer dimension of the carrier member 2. This may be done by adjusting the ratio of, for example, PEG di-acrylamide to sodium acrylate in embodiments wherein the expansile element 1 comprises a hydrogel. Alternatively, a relatively high dose of radiation could be used to cross-link the expansile element 1, thus limiting its expansion. Embodiments such as shown in **Fig. 8** are desirable when filling is necessary and it is desirable to have a substrate for tissue growth and proliferation that the expansile element 1 provides. In an embodiment used to treat cerebral aneurysms, this configuration could be used as a "filling" coil. In one embodiment, the expansile element 1 comprises a hydrogel incorporating a porosigen as previously described to provide a reticulated matrix to encourage cell growth and healing. Incorporating, for example, growth hormones or proteins in the expansile element 1 as previously described may further enhance the ability of the implant 11 to elicit a biological response.

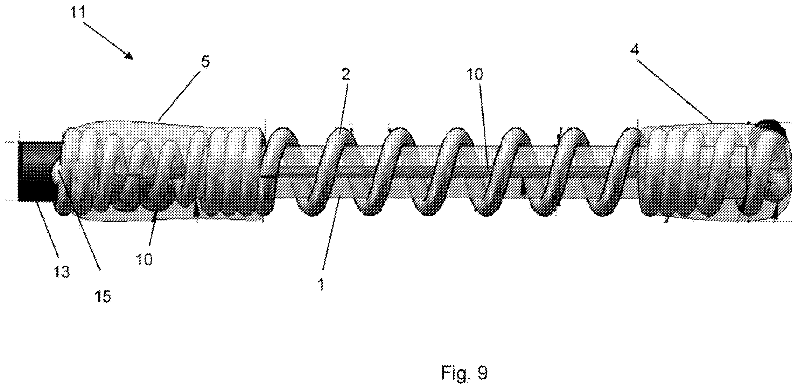

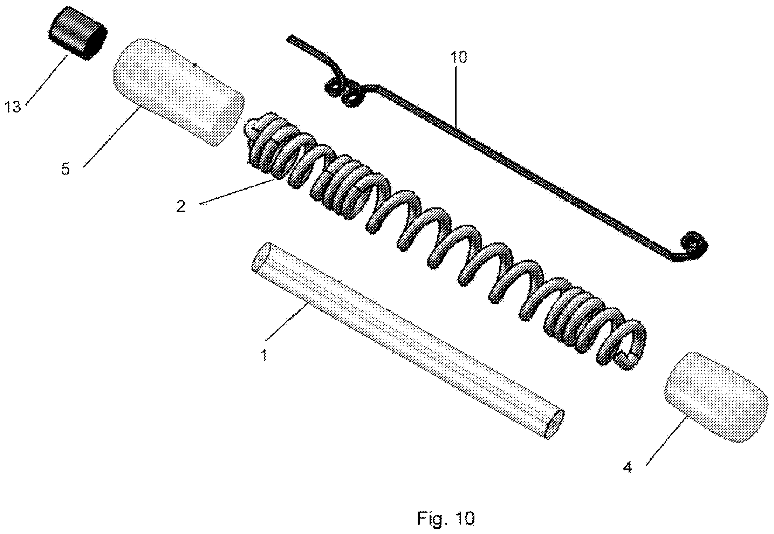

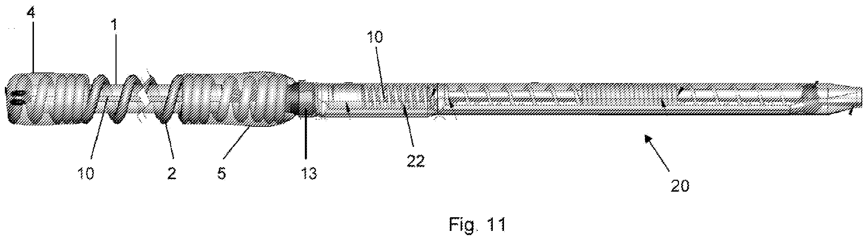

**Figs. 9-11** illustrate another preferred embodiment of an implant 11. This implant is generally similar to the previously described embodiments, including an expansile element 1 that is disposed within a carrier member 2. Additionally, a stretch resistant member 10 is positioned along a longitudinal axis of the expansile element 1 and attached to the distal end of the carrier member 2. The stretch resistant member 10 is preferably located within or partially surrounded by the expansile element 1. Preferably, the stretch resistant member 10 is wrapped around a proximal portion of the carrier member 2 and attached near a heater coil 22 within a distal end of a delivery device 20, shown in **Fig. 11****.**

As best seen in **Fig. 9****,** the proximal end of the carrier member 2 can include a coiled region having a smaller diameter than the other coiled regions of the member 2. This smaller diameter coiled region allows the stretch resistant member 10 to be wrapped around the member 2 without extending outwards past the diameter of the other coiled regions of the member 2. Additionally, a covering material 5 can be further positioned over the smaller diameter coiled region without the loops of the stretch resistant member 10 being exposed. Preferably, this covering material 5 is a laser, solder, adhesive, or melted hydrogel material.

As seen best in **Fig. 9****,** the spacing of the helical coils of the carrier member can vary along the length of the implant 11. For example, the coils can be located close to each other or touching each other near the proximal and distal ends while the center portion of the implant 11 can have coils with larger spaces between them. In other words, the gaps between the coils can be larger along most of the implant 11 and smaller near the ends of the implant 11.

In one embodiment, this implant 11 is created according to the following method. The expansile element 1 is created with hydrogel according to the previously described techniques in this specification. In one embodiment the expansile element 1 is formed in a polymerization tube between about 0.635 mm (0.025") and 0.8128 mm (0.032") ID. After polymerization, the polymerization tube is cut into segments that are dried under vacuum. Once all water has been removed from the hydrogel, the dried hydrogel is pushed out of the polymerization tube using a mandrel. The hydrogel is then washed in water three times, swelling the hydrogel and removing sodium chloride and unreacted monomers.

This expanded hydrogel is then skewered along its longitudinal axis (i.e., along an axis of its length) using a microcoil (or similar elongated tool). This skewing creates a pathway along the approximate center of the hydrogel filament so that a stretch resistant member 10 can be later threaded through. Next, the skewered hydrogel is acid treated by immersion into a hydrochloric acid solution, protonating the carboxylic acid moieties of the sodium acrylate component of the polymer network. The skewered hydrogel is finally washed in alcohol to remove residual acid and dried under a vacuum.

A gapped platinum coil is used for member 2, having an outer diameter ranging from about 0.3048 mm (0.012") to about 0.4572 mm (0.018") filar ranging from about 0.0381 mm (0.015") to about 0.0762 mm (0.0030") and gaps 7 ranging from about 0.0381 mm (0.0015") to about 0.1905 mm (0.0075"). In another embodiment the gaps 7 range from about 0.05715 mm (0.00225") to about 0.1905 mm (0.00750"). In one embodiment, this platinum coil has an outer diameter of about 0.3048 mm (0.012"), a filar of about 0.0508 mm (0.002") and a gap 7 of about 0.1016 mm (0.004"). In another embodiment, this platinum coil has an outer diameter of about 0.3175 mm (0.0125"), a filar of about 0.05715 mm (0.00225"), and a gap 7 of about 0.1143 mm (0.0045"). This gapped platinum coil is wound over a mandrel and heat-set into a secondary helical shape. The platinum coil is cut to a desired implant length and bonded to a coupling marker band or coupler 13 via soldering, welding or adhesive (e.g., weld 15 in Figure 9).

The coil used to skewer the hydrogel filament is removed, and an about .0022" polyolefin stretch-resistant thread for the stretch resistant member 10 is threaded through the filament along the pathway left by the coil. The hydrogel filament, which now has an outer diameter of between about 0.254 mm (0.010") to about 0.4572 mm (0.018") is stretched to an outer diameter between about 0.1524 mm (0.006") to about 0.3048 mm (0.012") and inserted into the gapped platinum body coil. While still under tension, the hydrogel filament is bonded to the body coil at both ends.

The stretch-resistant thread is knotted at the distal end of the platinum coil and wrapped around the open coil gaps at the proximal end (i.e., the end with coupler 13). Both ends of the implant 11 are covered with adhesive 4 and 5 to secure the stretch resistant member 10 and encapsulate the ends of the expansile element 1. Finally, the implant 11 is attached to a detachment pusher using the stretch resistant member 10 that protrudes from the proximal end of the implant 11.

During use of the implant 11 of this embodiment, the implant 11 is advanced via a detachment pusher 20 through a microcatheter (not shown). When the distal end of the microcatheter has reached a desired target area, the pusher 20 is advanced, thereby pushing the implant 11 out of the microcatheter. When the user wishes to detach the implant 11, a heater coil 22 is activated to break the stretch resistant member 10. Upon contact with the blood, the pH sensitive expansile element will expand to a final diameter between about 0.508 mm (0.020") and 0.889 mm (0.035") allowing the user about 5-10 minutes of working time.

In another embodiment, the implant 11 of Figure 9 includes a stretch-resistant member 10 composed of polyolefin and having an outer diameter of about 0.05588 mm (0.0022"). The expansile element 1 is composed of a hydrogel of about 48% PEG 8000 diacrylamide and 52% sodium acrylate. The member 2 is a gapped platinum coil having an outer diameter between about 0.3048 mm (0.012") and 0.508 mm (0.020") and more preferably about 0.3048 mm (0.012"). The member 2 has a filar between about 00381 mm (0.0015") and 0.127 mm (0.005") and more preferably about 0.0508 mm (0.002"). The gap between winds of the member 2 is preferably about 0.0762 mm (0.003")

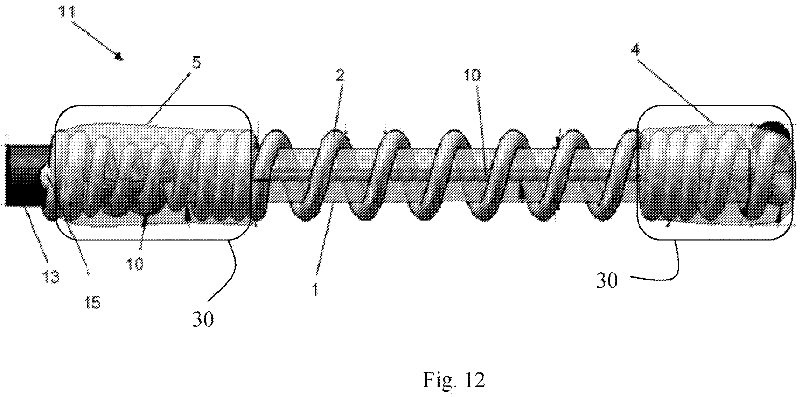

Figure 12 illustrates a preferred embodiment of an implant 11 similar to the previously described embodiment in which the gaps between winds of the member 2 are preferably between about 0.0508 mm (0.002") and 0.508 mm (0.020"). Additionally, the implant 11 contain one or more outer member 30 located at a proximal end of the implant 11, at a distal end of the implant, adjacent to the proximal or distal end of the implant, or at any combination of these locations. In the example of Figure 12, an outer member 30 is positioned at the proximal and distal ends of the implant 11.

In one example, the outer member 30 is preferably composed of platinum coil having a length between about 0.254 mm (0.010") and 3.048 mm (0.120") and more preferably between about 1.16 mm (0.040") and 2.032 mm (0.080"). The internal diameter of the outer member 30 is preferably between about 0.3048 mm (0.012") and 0.4318 mm (0.017") and more preferably between about 0.3048 mm (0.012") and 0.3175 mm (0.0125"). The wire of the outer member 30 preferably has a filar between about 0.0381 mm (0.0015") and about 0.0762 mm (0.003") and more preferably about 0.0381 mm (0.0015").

In another example, the outer member 30 is composed of a slotted tube having a length between about 0.254 mm (0.010") and 3.048 mm (0.120") and more preferably between about 1.016 mm (0.040") and 2.032 mm (0.080"). The internal diameter of the slotted tube is preferably between about 0.3048 mm (0.012") and 0.4318 mm (0.017") and more preferably between about 0.3048 mm (0.012") and 0.3175 mm (0.0125"). The thickness of the slotted tube is preferable between about 0.0254 mm (0.001") and 0.0762 mm (0.003") and more preferably about 0.0381 mm (0.0015").

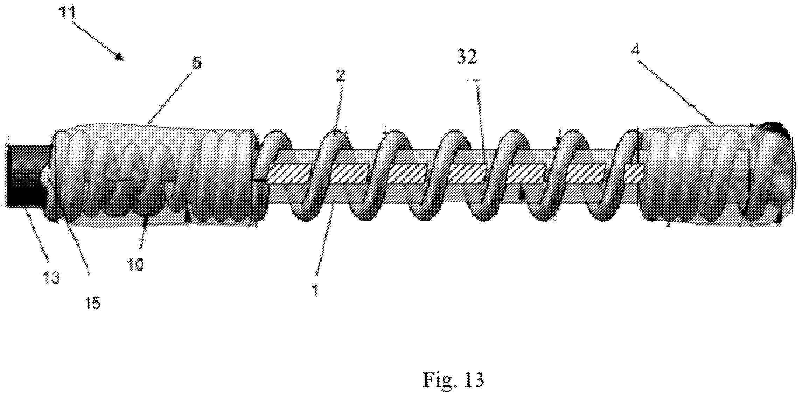

Figure 13 illustrates another preferred embodiment of the implant 11 that is generally similar to the previously described embodiment. However, this implant 11 further comprises a closed-wound platinum coil 32 disposed over the stretch-resistant member 10. Preferably, the stretch-resistant member 10 is composed of polyethylene and has an outer diameter of about 0.02286 mm (0.0009"). The closed-wound platinum coil 32 preferably has an outer diameter of about 0.1524 mm (0.006") and has a wire filar of about 0.0381 mm (0.0015"). The expansile element 1 is preferably composed of 48% PEG 8000 diacrylamide and 52% sodium acrylate. The member 2 is a gapped platinum coil having an outer diameter between about 0.3048 mm (0.012") and 0.508 mm (0.020") and more preferably between about 0.3556 mm (0.014") and 0.381 mm (0.015"). The member 2 has a filar between about 0.0381 mm (0.0015") and 0.127 mm (0.005") and more preferably about 0.0508 mm (0.002"). The gap between winds of the member 2 is preferably between about 0.0508 mm (0.002") and 0.508 mm (0.020") and more preferably 0.1016 mm (0.004").

Preferably, the implant 11 of Figure 13 is created by preparing expansile element 1 with hydrogel as previously described in this specification. Prior to the acid treatment, the hydrated hydrogel is skewered with a platinum coil 32. Preferably, the platinum coil 32 is heat-set into a predetermined helical shape with a defined pitch and diameter prior to skewering. A stiff and preferably platinum-based mandrel is inserted into the platinum coil 32 to provide support during further treatments and construction of the implant 11.

Following the acid treatment of the hydrogel, the mandrel is removed from within the platinum coil 32 and replaced by stretch-resistant member 10 (e.g., a polyolefin monofilament). Optionally, both the mandrel and the platinum coil 32 can also be removed and replaced by the stretch-resistant member 10. The member 2 (e.g., a gapped platinum coil) is placed over the resulting subassembly and is sized appropriately to allow little or no free space within the internal diameter of the member 2. The member 2 can optionally be wound and heat-set into a preliminary and preferably helical shape of a defined pitch and diameter prior to placing over the hydrogel and platinum coil 32.

Once the member 2 has been placed, it is bonded to the hydrogel using adhesives at proximal and distal ends (preferably UV-cured adhesives). At this point, outer members 30 can optionally be located and bonded at one or more ends of the implant 11. The stretch-resistant member 10 is then secured at both ends of the implant 11 and the implant 11 is coupled to an electrical detachment mechanism as described elsewhere in this specification.

In one embodiment a vaso-occlusive device comprises an expansile polymer element having an outer surface, a carrier member that covers at least a portion of the outer surface of the expansile polymer element, and wherein no carrier is disposed within the outer surface of the expansile element.

In another embodiment, a vaso-occlusive device comprises a coil having a lumen and a hydrogel polymer having an outer surface wherein the hydrogel polymer is disposed within the lumen of the coil and wherein the hydrogel polymer does not contain a coil within the outer surface of the hydrogel polymer.

In another embodiment, a vaso-occlusive device comprises a carrier member formed into a secondary configuration and an expansile element, wherein the expansile element is made from a polymer formulated to have sufficient softness that the expansile element will substantially take the shape of the secondary configuration formed into the carrier member without pre-treatment.

In another embodiment, a vaso-occlusive device comprises a carrier member formed into a secondary configuration and a substantially continuous length of hydrogel, wherein the device will substantially take the shape of the secondary configuration formed into the carrier member without pre-treatment.

In another embodiment, a vaso-occlusive device comprises a microcoil having an inner lumen and an expansile element disposed within the inner lumen. In this embodiment the expansile element comprises a hydrogel selected from the group consisting of acrylamide, poly(ethylene glycol), Pluronic, and poly(propylene oxide).

In another embodiment, a vaso-occlusive device comprises a coil and a hydrogel polymer disposed at least partially within the coil wherein the hydrogel has an initial length and wherein the hydrogel polymer has been stretched to a second length that is longer than the initial length.

In another embodiment, a vaso-occlusive device comprises an expansile element and a carrier member defining an inner lumen, wherein the expansile element is disposed within the inner lumen of the carrier member and wherein the expansile element has been stretched to a length sufficient to prevent a loop of the expansile element from protruding through the carrier member.

The examples disclosed herein also includes a method of manufacturing a medical device. The method comprises providing a carrier member having an inner lumen and an expansile element, inserting the expansile element into the inner lumen of the carrier member, and stretching the expansile element.

In another embodiment, a vaso-occlusive device comprises an expansile element encapsulated by a carrier element, wherein said expansile element is comprised substantially entirely and substantially uniformly of material having an expansile property.

In another embodiment, a vaso-occlusive device comprises a carrier element and an expansile element wherein the carrier element has a secondary shape that is different from its primary shape and wherein the expansile element is sufficiently flexible in a normal untreated state to conform with the secondary shape of the carrier.

In another embodiment, a vaso-occlusive device includes a carrier and an expansile element wherein the expansile element is fixed to the carrier in a manner such that the expansile element is in a stretched state along the carrier.

In another embodiment, a vaso-occlusive device includes a carrier having a plurality of gaps along the carrier and an expansile element positioned along an inside envelope of the carrier and wherein the expansion of the expansile element is controlled such that the expansile element expands into the gaps but not beyond the external envelope of the carrier.

In another embodiment, a vaso-occlusive device includes a carrier member and an expansile element wherein the expansile element is comprised of multiple strands extending along the carrier.

In another embodiment, a vaso-occlusive device includes a carrier and an expansile member wherein the carrier is a non-coiled cylindrically shaped structure and wherein said expansile member is disposed inside said carrier.

In another embodiment, a vaso-occlusive device includes a carrier and an expansile member and a stretch resistant member; said expansile member and said stretch resistant member being disposed in an internal region of the carrier and wherein the stretch resistant member is in tension on said carrier.

The examples disclosed herein also includes a method of treating a lesion within a body. The method comprises providing a vaso-occlusive device comprising a carrier member and an expansile element wherein the carrier member is formed into a secondary configuration that is approximately the same diameter as the lesion and inserting the vaso-occlusive device into the lesion.

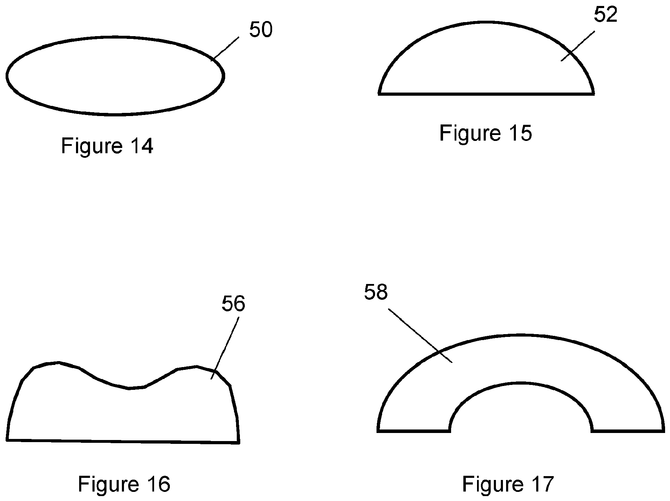

In one embodiment, the carrier member 2 is composed of a wire having a round cross sectional shape. In other preferred embodiments, the carrier member 2 of any of the embodiments shown in this specification (or variations thereof) can have a non-round cross sectional-shape (e.g., a cross-sectional shape with a non-uniform or varying diameter at different angles or locations across the cross section). For example, Figure 14 illustrates a wire 50 with generally oval cross sectional shape, Figure 15 illustrates a wire 52 with a half circle or "D" cross sectional shape, Figure 16 illustrates a wire 56 with a "double D" shape, a half-circle with a center depression, or a channeled half-circle cross sectional shape, and Figure 17 illustrates a U or an arc cross-sectional shape.

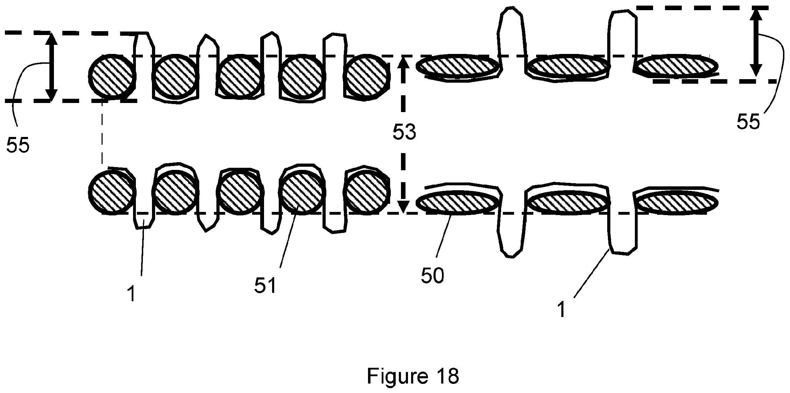

These non-round cross sectional wire shapes can provide performance characteristics that can be desirable in some uses. One such characteristic can be seen in Figure 18, which compares a carrier member 2 with a round cross sectional wire 51 to that of a wire 50 with an oval cross section. The expansile element 1 can typically extend a limited distance beyond the inner diameter of the implant 11 (shown as distance 55 in Figure 18). In some configurations of the device 11 using hydrogel, the expansile element 1 can expand between 0.1016 mm (0.004") - 0.1524 mm (0.006") beyond the implant's inner diameter before grating or breaking off.

By decreasing the thickness of the wire 50, the expansile element 1 can expand a greater distance beyond the outer diameter 53 of the implant 11. Hence, the implant 11, as a whole, can swell or expand to a greater diameter than an implant having a round cross sectional wire 51 but similar width, coil spacing and other performance characteristics.

In a more specific example, the wire 51 has a diameter of 0.3048 mm (0.012") and forms an implant with an inner diameter of 0.2032 mm (0.008") and an outer diameter of 0.3048 mm (0.012"). The wire 50 has a cross section of 0.254 mm x 0.1016 mm (0.001" x 0.004") with an inner diameter of 0.254 mm (0.010") and an outer diameter of 0.3048 mm (0.012"). The expansion limit from the inner diameter of the implant is about the same for each example (e.g., between 0.1016-0.1524mm (0.04"-0.006"), which therefore allows about 0.0508 mm (0.002") of additional expansion diameter on the implant using wire 50.

It should be noted that reducing the diameter of wire 51 to a similar thickness as wire 50 may achieve similar expansion diameters of the expansile element 1, but may sacrifice other important performance characteristics. For example, decreasing the wire mass also decreases the "pushability" of the implant 11. In other words, reducing the wire mass can decrease the column strength of the coiled implant and therefore increase the likelihood of the implant kinking, being damaged or similar complications. In another example, reducing the wire mass may also reduce radiopacity of the implant and therefore may be difficult to view during a procedure.

In contrast, the non-round wires 50, 52, 56 and 58 can provide a similar column strength and radiopacity as compared with a similarly-massed wire 51 while increasing the inner diameter of the implant 11. Further, some non-round cross sectional wire shapes may provide a higher column strength and radiopacity as compared with a similarly-massed round cross section wires 51.

Additionally, the non-round wires 50, 52, 56 and 58 can provide a frictional drag that is similar to that of the round wire 51, especially if the non-round wire has a non-flat surface oriented outward from the implant 11. For example, wires 50, 52, 56 and 58 all include non-flat or rounded surfaces that, when oriented outward from the implant 11, can provide a similar amount of surface area that can contact an inner lumen of a delivery catheter as compared with a similarly-massed wire 51.

The distal end of an implant tends to contact a patient's blood for the longest period of time during a procedure. Often, this distal exposure results from blood partially entering the catheter during advancement to a target area and from the distal end being pushed out from the catheter first (and therefore being fully exposed for the longest time). Since expansion of the expansile material 1 can be triggered by exposure to blood over a period of time, the expansile material near the distal end of an implant can otherwise fully expand first and thereby limit a user's ability to retract and reposition the device inside a patient.

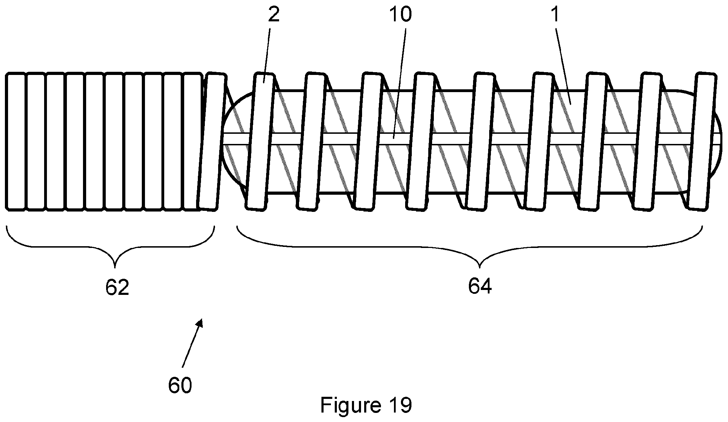

The implant 60 of Figure 19 can reduce premature expansion of its distal end (i.e., increase its exposure time for fully expanding its expansile material). Specifically, the implant 60 includes a distal region 62 in which its loops are "close-wound" or positioned immediately in contact with adjacent loops so as to be gapless (as opposed to the "open-wound" or gapped configuration of proximal region 64). This configuration can reduce the amount of expansile material 1 near the distal end that is contacted by blood prior to full deployment of the implant 60 from a catheter.

Initial blood exposure to distal portions of the expansile element 1 is further reduced by terminating the distal end of the expansile element proximal to the region 62. In other words, the expansile element 1 is located only within the proximal, open-wound region 64. In this regard, blood exposure to the distal portion of the expansile element 1 can be reduced, allowing a user more time to position or reposition the implant 60 in the patient before such actions are limited by the expansion of the expansile element 1.

In one example, the proximal region 64 has a gap size between its coils between about 0.0254 mm (0.001 inches) and about 0.254 mm (0.010 inches). In a more specific example, the gap size of the proximal region 64 is about 0.0762 mm (0.003 inches).

In another example, the carrier member 2 is a non-circular wire having a filar width between about 0.0254 mm (0.001 inches) and about 0.254 mm (0.010 inches) and a filar thickness between about 0.0127 mm (0.0005 inches) to about 0.2032 mm (0.008 inches). In a more specific examples, the carrier member 2 has a filar width of about 0.1016 mm (0.0040 inches) and a filar thickness of about 0.04572 mm (0.0018 inches).

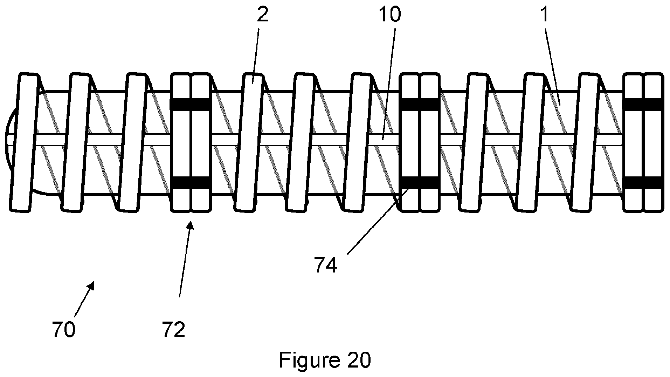

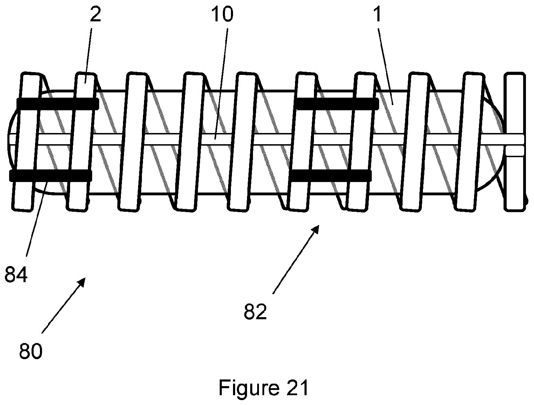

In another example, the implant 60 has an outer diameter between about 0.2032 mm (0.008 inches) and about 0.6604 mm (0.0026 inches). While the previously described, example dimensions are applicable to the implant 60, it should be understood that these dimensions are also possible for any of the other embodiments discussed in this specification, especially those shown in Figures 20 and 21.

Figure 20 illustrates a portion of an implant 70 that is generally similar to the devices previously described in this embodiment. However, the implant 70 includes a plurality of connected or fused loop regions 72 that are located along the length of the implant 70. Hence, the implant 70 alternates between regions of open-wound or gapped loops and regions of directly connected loops 72. These regions 72 can effectively increase the overall spring constant of the implant 70 (as compared with a similar implant without the regions 72), thereby improving the implant's "pushability" or ease of being pushed out a catheter without buckling or similar undesirable movement.

As seen in Figure 20, each region can include two connected loops. Alternately, any number of loops can be connected together, such as between 2 and 6 loops. The loops can be connected to each other via a solder joint 74, glue, welding, or similar bonding techniques. The regions 72 can be located at any regular intervals or distances from each other, such as the three loop interval shown in Figure 20. Alternately, the implant 70 can have areas of different spacing between regions 72 (e.g., discretely different regions or progressively increasing/decreasing distances along the length of the implant 70 towards its distal end). For example, the distal and proximal ends may have regions 72 that are closer together than a middle region. In another example, the length between regions 72 may increase from the proximal to distal end of the implant 70.

Figure 21 illustrates a similar variation of implant 70. However, the implant 80 includes fixed-distance regions 82 in which elongated fixture members 84 maintain adjacent loops at a predetermined distance from each other. These fixture members 84 can be soldered, welded, adhered or bonded to the adjacent loops so as to prevent relative movement between the loops of the region 82. The fixed-distance regions 82 are preferably proceeded and followed by the non-fixed regions (i.e., distally and proximally). While only two connected loops are shown as part of region 82, any number of loops can be connected (e.g., 3, 4, 5, 6, 7, 8, 9, or 10). Preferably, the loops of region 82 have a spacing from each other similar to (i.e., about the same as) the other loops of the implant 80, such that all of the implant's loops have a relatively uniform spacing. Alternately, the loops of the region 82 may have a different spacing than loops not touching fixture member 82 (i.e., larger or smaller).

As with the previous implant 70, the regions 82 can be spaced in a number of different configurations along the length of the device 80. For example, the regions 82 can be located along only a part of the device's length, in regularly increasing/decreasing intervals or in discrete segments of different spacing. Again, these fixture members 84 can effectively increase the overall spring constant of the implant 80 (as compared with a similar device without the regions 82), thereby improving the device's "pushability" or ease of being pushed out a catheter without buckling or similar undesirable movement.

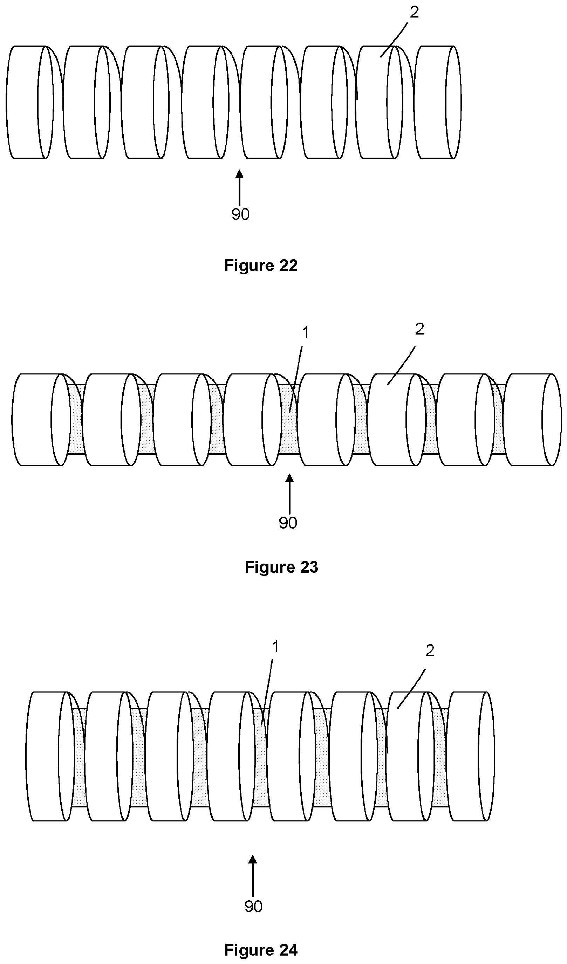

Figures 22-24 illustrate an alternative embodiment of an implant 90. The helical carrier member 2 has an initial, unconstrained diameter (for instance, a coil which is heat set into an initial helical shape) as is shown in Figure 22. The expansile element 1 is placed within the interior of the carrier member 2 and secured to the carrier 2 via UV adhesive, or other methods previously described. The expansile element is placed under tension, such as by stretching, in order to reduce the radial profile of the element which correspondingly reduces the radial profile of the attached carrier member, which is secured to the expansile element, as shown in Figure 23. When the implant is placed in a physiologically appropriate medium (i.e. blood or phosphate buffer solution), the expansile element swells; causing the constrained carrier diameter to swell and returned to its unconstrained state, as shown in Figure 24.

Different sections of the carrier could be constrained at different levels of tension in order to form variable constrained diameters. For example, the distal or proximal end of the carrier could be constrained at a higher level of tension than the other end, or the tension level could vary axially throughout the carrier member. This would allow for a variable constrained, and variable expanded/unconstrained implant shape which may be useful for space filling in more challenging geometrical shapes.

In one example, in order to help maximize the amount of swelling of the expansile element, said element is preferably not stretched to a significant degree prior to being secured to the carrier member. Certain previous embodiments described stretching the expansile element prior to adhesion to the carrier member in order, for example, to prevent folds from forming during expansion. However, stretching to a significant degree could limit the amount of expansion of the element, once it is exposed to the physiologically appropriate medium.

This embodiment may function effectively as a framing coil, where placement of the implant is easier due to the constrained, smaller size of the implant, and where the swelled, unconstrained larger size of the implant over time allows for a more secure placement around the periphery of the aneurysm, or vessel deformation. This embodiment can also function effectively as a filling or finishing coil, where the constrained implant diameter allows easier placement of the implant (especially in tight spaces) and the swelled, unconstrained diameter will maximize space filling.

Although preferred embodiments of the invention have been described in this specification and the accompanying drawings, it will be appreciated that a number of variations and modifications may suggest themselves to those skilled in the pertinent arts. Thus, the scope of the present invention is not limited to the specific embodiments and examples described herein, but should be deemed to encompass alternative embodiments and equivalents. The invention is defined in the claims.

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain embodiments of this invention are described herein, including the best mode known to the inventors for carrying out the invention. Of course, variations on these described embodiments will become apparent to those of ordinary skill in the art upon reading the foregoing description. The inventor expects skilled artisans to employ such variations as appropriate, and the inventors intend for the invention to be practiced otherwise than specifically described herein. Accordingly, this invention includes all modifications and equivalents of the subject matter recited in the claims appended hereto as permitted by applicable law. Moreover, any combination of the above-described elements in all possible variations thereof is encompassed by the claims unless otherwise indicated herein or otherwise clearly contradicted by context.

Furthermore, numerous references have been made to patents and printed publications throughout this specification. Each of the above-cited references and printed publications are individually incorporated herein by reference in their entirety.

In closing, it is to be understood that the embodiments of the invention disclosed herein are illustrative of the principles of the present invention. Other modifications that may be employed are within the scope of the claims. Thus, by way of example, but not of limitation, alternative configurations of the present invention may be utilized in accordance with the teachings herein. Accordingly, the present invention is not limited to that precisely as shown and described but only by the wording of the claims.

## Claims

1. A vaso-occlusive device for body cavities comprising:
a helical carrier member (2) formed from wire (50, 52, 56, 58) having a non-circular cross-sectional shape and being oriented such that a non-flat surface of said wire (50, 52, 56, 58) is oriented outward from said vaso-occlusive device; **characterised in that** the vasco-occlusive device comprises
an expansile member (1) disposed within said helical carrier member (2); wherein
the helical carrier member (2) incorporates at least one gap (7) that is dimensioned to allow the expansile member (1) to expand through said gap (7) and the expansile member is configured to expand through said gap (7).

2. The vaso-occlusive device of claim 1, wherein said non-circular cross-sectional shape has a diameter that varies in length at different locations.

3. The vaso-occlusive device of claim 1, wherein said non-circular cross-sectional shape is oval.

4. The vaso-occlusive device of claim 1, wherein said non-circular cross-sectional shape is a half-circle shape.

5. The vaso-occlusive device of claim 1, wherein said non-circular cross-sectional shape is a half-circle shape having a center depression.

6. The vaso-occlusive device of claim 1, wherein said non-circular cross-sectional shape is a double D shape.

7. The vaso-occlusive device of claim 1, wherein said non-circular cross-sectional shape is a half-circle shape with a middle channel.

8. The vaso-occlusive device of claim 1, wherein said non-circular cross-sectional shape is an arc shape.

9. The vaso-occlusive device of claim 1, wherein said non-circular cross-sectional shape is a U shape.

## Patentansprüche

1. Vasookklusive Vorrichtung für Körperhöhlen, umfassend:
spiralförmiges Trägerbauteil (2), das aus Draht (50, 52, 56, 58) geformt ist, das eine nicht kreisförmige Querschnittsflächenform hat, und so ausgerichtet ist, dass eine nicht flache Oberfläche des Drahtes (50, 52, 56, 58) von der vasookklusiven Vorrichtung auswärts ausgerichtet ist, **dadurch gekennzeichnet, dass** die vasookklusive Vorrichtung umfasst:
sich ausdehnendes Bauteil (1), das in dem spiralförmigen Trägerbauteil (2) angeordnet ist; wobei das spiralförmige Trägerbauteil (2) mindestens eine Lücke (7) umfasst, die so dimensioniert ist, dass sie die Ausdehnung des sich ausdehnenden Bauteils (1) durch die Lücke (7) hindurch erlaubt, und dass das sich ausdehnende Bauteil so konfiguriert ist, dass es sich durch die Lücke (7) hindurch ausdehnt.

2. Vasookklusive Vorrichtung gemäß Anspruch 1, wobei die nicht kreisförmige Querschnittsflächenform einen Durchmesser aufweist, der an unterschiedlichen Stellen in seiner Länge variiert.

3. Vasookklusive Vorrichtung gemäß Anspruch 1, wobei die nicht kreisförmige Querschnittsflächenform oval ist.

4. Vasookklusive Vorrichtung gemäß Anspruch 1, wobei es sich bei der nicht kreisförmigen Querschnittsflächenform um eine Halbkreisform handelt.

5. Vasookklusive Vorrichtung gemäß Anspruch 1, wobei es sich bei der nicht kreisförmigen Querschnittsflächenform um eine Halbkreisform mit einer zentralen Vertiefung handelt.

6. Vasookklusive Vorrichtung gemäß Anspruch 1, wobei es sich bei der nicht kreisförmigen Querschnittsflächenform um eine doppelte D-Form handelt.

7. Vasookklusive Vorrichtung gemäß Anspruch 1, wobei es sich bei der nicht kreisförmigen Querschnittsflächenform um eine Halbkreisform mit einem Mittelkanal handelt.

8. Vasookklusive Vorrichtung gemäß Anspruch 1, wobei es sich bei der nicht kreisförmigen Querschnittsflächenform um eine Bogenform handelt.

9. Vasookklusive Vorrichtung gemäß Anspruch 1, wobei es sich bei der nicht kreisförmigen Querschnittsflächenform um eine U-Form handelt.

## Revendications

1. Dispositif d'occlusion vasculaire pour des cavités de corps comprenant :
un élément de support hélicoïdal (2) formé à partir d'un fil (50, 52, 56, 58) ayant une forme de section transversale non circulaire et étant orienté de sorte qu'une surface non plate dudit fil (50, 52, 56, 58) soit orientée vers l'extérieur par rapport audit dispositif d'occlusion vasculaire ;
**caractérisé en ce que** le dispositif d'occlusion vasculaire comprend
un élément extensible (1) disposé dans ledit élément de support hélicoïdal (2) ; dans lequel
l'élément de support hélicoïdal (2) incorpore au moins un espace (7) qui est dimensionné pour permettre à l'élément extensible (1) de s'étendre à travers ledit espace (7) et l'élément extensible est configuré pour s'étendre à travers ledit espace (7).

2. Dispositif d'occlusion vasculaire selon la revendication 1, dans lequel ladite forme de section transversale non circulaire a un diamètre qui varie en longueur à différentes positions.

3. Dispositif d'occlusion vasculaire selon la revendication 1, dans lequel ladite forme de section transversale non circulaire est ovale.

4. Dispositif d'occlusion vasculaire selon la revendication 1, dans lequel ladite forme de section transversale non circulaire est une forme de demi-cercle.

5. Dispositif d'occlusion vasculaire selon la revendication 1, dans lequel ladite forme de section transversale non circulaire est une forme de demi-cercle ayant une dépression centrale.

6. Dispositif d'occlusion vasculaire selon la revendication 1, dans lequel ladite forme de section transversale non circulaire est une forme de D double.

7. Dispositif d'occlusion vasculaire selon la revendication 1, dans lequel ladite forme de section transversale non circulaire est une forme de demi-cercle avec un canal intermédiaire.

8. Dispositif d'occlusion vasculaire selon la revendication 1, dans lequel ladite forme de section transversale circulaire est une forme d'arc.

9. Dispositif d'occlusion vasculaire selon la revendication 1, dans lequel ladite forme de section transversale circulaire est une forme de U.
